# EUROPEAN PATENT SPECIFICATION

(11) **EP 3 028 706 B1**
(45) Date of publication and mention of the grant of the patent: **19.08.2020**
(21) Application number: 14831491.7
(22) Date of filing: 24.07.2014
(51) Int. Cl.: A61K 9/24, A61K 31/216, A61K 31/573, A61P 29/00

(54) **PHARMACEUTICAL COMPOSITION WITH ANTIINFLAMMATORY AGENTS AND PRODUCTION PROCESS**
PHARMAZEUTISCHE ZUSAMMENSETZUNG MIT ENTZÜNDUNGSHEMMERN UND HERSTELLUNGSVERFAHREN
COMPOSITION PHARMACEUTIQUE COMPRENANT DES AGENTS ANTI-INFLAMMATOIRES ET PROCÉDÉ DE PRODUCTION

(30) Priority: 02.08.2013 MX 2013008992
(43) Date of publication of application: 08.06.2016
(73) Proprietor: Laboratorio Raam de Sahuayo, S.a. de C.V., 59000 Sahuayo, Michoacán (MX)
(72) Inventor: AMEZCUA AMEZCUA, Federico, 59000 Sahuayo, MICH (MX); COVARRUBIAS PINEDO, Amador, 45010 Zapopan, JAL (MX)
(74) Representative: Balder IP Law, S.L.
(86) International application number: PCT/MX2014/000117
(87) International publication number: WO 2015/016698

(56) References cited:
- EP-A2- 2 583 674
- WO-A1-2010/090371
- WO-A2-00/24388
- GB-A- 874 586
- US-A1- 2008 187 586
- US-B1- 6 423 697

## Description

### Field of the invention

The present invention relates to novel pharmaceutical compositions comprising a three-phase release system having at least three layers to form at least one oral dosage unit, consisting of at least two outer layers, with at least one core spatially located. The layers may have a delayed- or extended-release system, or an immediate-release system, and contain at least one corticosteroid agent, at least one non-steroidal anti-inflammatory agent, and at least one pharmaceutically acceptable excipient. The compositions are useful for the treatment of body pain and inflammation of affected areas. The invention also relates to a process for the preparation of the novel pharmaceutical compositions.

### BACKGROUND

The adrenal glands are multifunctional endocrine organs secreting various steroids, which can be divided into corticosteroids based on a 21-carbon nucleus and sex steroids, mainly androgens, based on a 19-carbon nucleus. Corticosteroids are traditionally divided by their metabolic activities into those with glucocorticoid actions, of which cortisol (hydrocortisone) is the most important endogenous example, and those with electrolyte regulation, which primarily are mineralocorticoids, of which aldosterone is the most important. Glucocorticoids are the ones who have more therapeutic applications.

### (Sweetman, S. 2002)

Numerous experimental and clinical observations have shown that the adrenal glands are essential for life. Their secretions play a wide variety of physiological functions such as regulation of blood glucose, protein turnover, metabolism of fat, balance of sodium, potassium and calcium, modulation of tissue response to injuries or infections and, above all, survival to any stress. (Berne, L. 1998)

Glucocorticoids are potent suppressors of inflammation. These can prevent or suppress inflammation in response to multiple provoking phenomena, including radiant, mechanical, chemical, infectious, and immune stimuli. Although the use of glucocorticoids as anti-inflammatory does not attack the root cause of the disease, the suppression of inflammation has enormous clinical utility, and has led these compounds to be among those most frequently prescribed. Similarly, glucocorticoids are immensely useful for treating diseases originated by undesirable immune reactions, including diseases that occur predominantly by humoral immunity, like hives. (Goodman and Gilman. 2003)

The effects of corticosteroids include: abnormal metabolism of carbohydrates, proteins and lipids; conservation of fluid and electrolyte balance, and preservation of normal function of the cardiovascular and immune systems, kidneys and striated muscle, as well as the endocrine and nervous systems. In addition, corticosteroids allow the body to withstand circumstances that generate stress, as harmful stimuli and environmental changes. Corticoids also have an effect at the level of absorption and excretion of calcium, resulting in a decrease in reserves of calcium in the body. (Goodman and Gilman, Sweetman, S. 2003, 2002)

Corticosteroids are grouped according to their relative potencies of retaining sodium, actions on carbohydrate metabolism (i.e., glycogen deposition in the liver and glycogenesis) and anti-inflammatory effects as shown in Table 1.

**Table 1. Relative potencies and equivalent doses of representative corticosteroids.**

| Compound | Anti-inflammatory potency | Potency to retain Na+ | Duration of action | Dose equivalents ◆mg |
|---|---|---|---|---|
| Cortisol | 1 | 1 | B | 20 |
| Cortisone | 0.8 | 0.8 | B | 25 |
| Fludrocortisone | 10 | 125 | I | ** |
| Prednisone | 4 | 0.8 | I | 5 |
| Prednisolone | 4 | 0.8 | I | 5 |
| 6α-methyl prednisolone | 5 | 0.5 | I | 4 |
| Tiamcinolone | 5 | 0 | I | 4 |
| Betamethasone | 25 | 0 | p | 0.75 |
| Dexamethasone | 25 | 0 | p | 0.75 |

| | | | | |
|---|---|---|---|---|
| **B, short** (Biological half-life of 8-12 h); **I, intermediate** (Biological half-life of 12-36 h); **P, prolonged** (Biological half-life of 36 to 72 h). ◆ These dose relationships apply only to the oral or intravenous use, since the powers of glucocorticoids may differ long after intramuscular or intra-articular administration. ** This compound is not used for obtaining glucocorticoid effects. | | | | |

The anti-inflammatory and immunosuppressive properties of glucocorticoids make them invaluable therapeutic agents in numerous diseases, but do not attack the underlying cause of the disease.

Glucocorticoids influence the traffic of circulating leukocytes and immune accessory cells. They suppress immune activation of these cells, inhibit the production of cytokines and other mediators of inflammation, and produce resistance to cytokines. Reduce or prevent tissue response to inflammatory processes, thereby reducing the development of inflammation symptoms without affecting the underlying cause. These drugs inhibit the accumulation of inflammatory cells, including leukocytes and macrophages. They also inhibit phagocytosis, the release of lysosomal enzyme, and the synthesis and/or release of chemical mediators of inflammation.

Hydrocortisone and many congeners, including synthetic analogs, are effective when administered orally. Most of the corticosteroids that are used systemically are hydroxy (alcohol) compounds. They are relatively insoluble in water and the sodium salt of phosphate or succinate ester is generally used to provide water-soluble forms for solutions. These esters are hydrolyzed easily in the body.

Betamethasone has a very important anti-inflammatory property; it intervenes on phospholipase A2 by blocking the production of arachidonic acid, avoiding the cascade of chemical mediators involved in the inflammatory process.

Concomitant use of glucocorticoids with non-steroidal anti-inflammatory drugs (NSAIDs) can provide an additive therapeutic effect, which allows to reduce the dose of glucocorticoids.

Non-steroidal anti-inflammatory drugs (NSAIDs) are a group of drugs that have anti-inflammatory, analgesic and antipyretic action, which act by inhibiting the activity of cyclooxygenase enzymes that convert arachidonic acid found in cell membranes in unstable cyclic endoperoxydes that are transformed into prostaglandins (PGs) and thromboxanes, which are involved in the pathogenic mechanisms of inflammation, pain and fever (Feria, 1997).

Aceclofenac is a non-steroidal anti-inflammatory drug (NSAID), derived from aryl acetic acid, structurally related to diclofenac. It is a potent inhibitor of the cyclooxygenase enzyme, which is involved in the production of prostaglandins.

The pharmacokinetic properties of aceclofenac have been studied after oral administration of single or multiple doses in healthy young and elderly volunteers, as well as in patients with osteoarthritis.

Following oral administration of a dose of 100 mg to healthy young volunteers, maximum plasma concentration (Cmax) of aceclofenac at 6.8 to 8.9 mg/L was reached between 1.4 to 2 hours (Tmax). The duration of treatment had only a small effect on the pharmacokinetic parameters of aceclofenac, which were similar after a single dose or multiple doses in young or elderly volunteers. The presence of food did not modify the pharmacokinetics of aceclofenac, except tmax, from 2.0 to 3.5 hours.

The average aceclofenac concentration in synovial fluid reached half that of plasma after treatment with 225 mg of aceclofenac daily for 6 days.

It is metabolized mainly into 4-hydroxi-aceclofenac; the other metabolites, diclofenac and 4-hydroxy-diclofenac, only represent 5% of an administered dose.

Approximately 70% of the dose is excreted in urine, mainly as aceclofenac glucuronide and diclofenac, and its hydroxy metabolites; the rest is excreted in feces. After a single dose of 100 mg aceclofenac, the elimination half-life is 6.2 hours.

Concomitant administration of an oral single dose of glucocorticoid and a non-steroidal anti-inflammatory is essential for the treatment and/or control of inflammation and/or body aches and/or diseases related thereto.

The main applications are: For the treatment of acute pain associated with headache, dental procedures, minor surgery, with oral administration, rapid absorption and short half-life. Also chronic pain, fever, osteoarthritis, rheumatoid arthritis, gout, primary dysmenorrhea, cardiovascular disease, Alzheimer's disease, tendonitis, myalgia, neuralgia, acute rheumatic fever, diseases associated with platelet aggregation (coronary artery disease, acute myocardial infarction, transient cerebral ischemia, etc.), among other diseases.

In the state of the art are described patents protecting a kit and method of administration of aceclofenac esters and betamethasone esters in aerosol (US 7,078,019); a method of manufacturing of a solid dispersion comprising dissolving a selected water-insoluble drug of aceclofenac and betamethasone and a substitute of cyclodextrin in an organic solvent and drying under reduced pressure these dispersions to improve the speed of dissolution of drugs and maximize its bioavailability (WO03043602); a therapeutic composition and treatment methods, containing betamethasone, aceclofenac, an immunosuppressant, and an IRM component (WO2005055932); a pharmaceutical composition containing betamethasone, aceclofenac and other compounds to inhibit COX and LOX, its process of preparation and method of use against inflammation and/or pain, and/or disorders associated (WO2007072503); a pharmaceutical composition containing azelastine or betamethasone and aceclofenac for transnasal or ocular administration (WO2006058022); a device for administering aceclofenac or betamethasone to respiratory system. A two-layer tablet comprising an inner core comprising a steroid and a coating comprising acetylsalicylic acid, useful for the treatment of pain and inflammation has been disclosed (GB874586).

Body pain with inflammation of the affected areas is a very common public health problem, which over time can become a risk factor that may lead to a poor quality of life and restrict physical activity in general. For this purpose, an improvement and/or control of such ailments can be achieved by means of a treatment, managing to have a significant impact at the population level.

### OBJECT OF THE INVENTION

Disclosed herein are novel pharmaceutical compositions with a three-phase release system, consisting of at least three layers, with at least one spatially located nucleus, which may have a delayed- or extended-release system, or an immediate-release system, contain at least one corticosteroid agent, at least one non-steroidal anti-inflammatory agent, and at least one pharmaceutically acceptable excipient, wherein said active pharmaceutical ingredients provide synergistic clinical effects at the time they are released from the formulation in order to achieve optimized therapeutic effects.

The present invention relates to a pharmaceutical oral tablet, characterized by comprising a three-phase release system with three layers: a) a core, b) an under layer, and c) a top layer, each one comprising a pharmaceutically acceptable carrier, wherein the core comprises one corticosteroid agent, preferably betamethasone, or pharmaceutically acceptable salts thereof; the under layer comprises one non-steroidal anti-inflammatory agent, preferably aceclofenac, or pharmaceutically acceptable salts thereof; the top layer comprises one non-steroidal anti-inflammatory agent, preferably aceclofenac, or pharmaceutically acceptable salts thereof, and wherein the core has a delayed-release system or an extended release system; the under layer covers at least half of the central core and has an immediate-release system; the top layer completely covers the central core and has an extended-release system or delayed-release system; the under layer and the top layer have different release systems, and tablet is formulated in at least one dosage unit and is useful for the treatment of body pain and inflammation of affected areas. The invention also relates to the process for the preparation of this pharmaceutical oral tablet.

The invention is defined by the claims. Any subject-matter falling outside the scope of the claims is provided for information purposes only.

### DETAILED DESCRIPTION OF THE INVENTION

The present invention relates to novel solid pharmaceutical compositions obtained using a new production process, comprising a three-phase release system capable of reducing side effects and/or dependency from treatment, among some other benefits, when they are in the same composition and different from those obtained by the independent administration of pharmaceutically active agents.
The administration of these novel solid pharmaceutical compositions, which have a three-phase release system, allows to have benefits such as increased efficiency of therapeutic effect and/or pharmaceutically active agents in lower concentrations and/or lower dose, and/or more extended times for administration and/or lower risk of some liver damage.

Such solid pharmaceutical compositions are composed of at least three layers to form an oral dosage unit, wherein at least each layer contains at least one pharmaceutically active agent belonging to group a) corticosteroid or at least one agent belonging to group b) non-steroidal anti-inflammatory drug, plus one or more pharmaceutically acceptable excipients. The pharmaceutically active agents, when administered orally in a single dosage form concomitantly, cause a better therapeutic effect.

The present invention is characterized by comprising a novel system of distribution of pharmaceutically active agents contained in an oral dosage unit with a three-phase release system. The release system three-phase because the oral dosage unit is comprised of at least three layers, wherein the first layer consists of a core located in the center of the oral dose unit; the second underlayer covers at least half of the central core; the third upper layer completely covers the central core and gives the final form of the oral dosage unit.

According to the above, the pharmaceutically active agent of the corticosteroid group, and/or salts, and pharmaceutically acceptable excipients thereof are present in the first layer; the pharmaceutically active agent of the non-steroidal anti-inflammatory group, and/or their salts and pharmaceutically acceptable excipients are present in the second and third layers.

The three-phase release system of the solid dosage unit was determined from the behavior and/or pharmacokinetic characteristics of these pharmaceutically active agents when administered in an oral dosage; also, the times for release of each pharmaceutically active agent are adjusted primarily to reduce side effects that they can cause and/ or adverse reactions associated with the administration thereof, in order to obtain an effective, efficient and safe pharmaceutical composition with respect to those reported in the state of the art.

The three-phase release system for pharmaceutically active agents of the dosage unit is useful for the treatment of body pain and inflammation of affected areas.

The three-phase release system contains the synergistic combination of such pharmaceutically active agents contained in at least one solid dosage unit for oral administration composed of at least three layers as follows: the first layer or core comprising at least a corticosteroid or pharmaceutically acceptable salts thereof with a delayed-release system or an extended-release system, and at least one pharmaceutically acceptable excipient; a second underlayer comprising at least one non-steroidal anti-inflammatory agent or pharmaceutically acceptable salts thereof with an immediate-release system, and at least one pharmaceutically acceptable excipient; and a third top layer comprising at least one non-steroidal anti-inflammatory agent or pharmaceutically acceptable salts thereof with an extended-release system or a delayed-release system, and at least one pharmaceutically acceptable excipient. Preferably, for the object of the present invention is used at least one core containing a corticosteroid agent or pharmaceutically acceptable salts thereof having a delayed release system; at least one underlayer containing a non-steroidal anti-inflammatory agent or pharmaceutically acceptable salts thereof having an immediate-release system; and at least one top layer containing a non-steroidal anti-inflammatory agent or pharmaceutically acceptable salts thereof having an extended release system, in order to avoid antagonistic competition for cytochrome P-450 in the liver, allowing to have the synergistic effect of the two pharmaceutically active agents.

Novel compositions of this invention are characterized because the nucleus comprises a) at least one pharmaceutically active corticosteroid or its pharmaceutically acceptable salts, i) at least one diluent, and/or ii) at least one binding agent, and/or iii) at least one release polymer, and/or iv) at least one lubricant, and/or vii) at least one solvent; the underlayer comprises b) at least one pharmaceutically active non-steroidal anti-inflammatory agent or its pharmaceutically acceptable salts, i) at least one diluent, and/or ii) at least one binding agent, and/or iii *) at least one disintegrating agent, and/or iv) at least one lubricant, and/or v) at least one solvent; the top layer comprises c) at least one pharmaceutically active non-steroidal anti-inflammatory agent or its pharmaceutically acceptable salts, i) at least one diluent, and/or (ii) at least one binding agent, and/or (iii) at least one release polymer, and/or (iv) at least one lubricant, and/or (v) at least one solvent.

In addition, the layers forming the oral dosage unit may or may not contain other pharmaceutically acceptable excipients selected from: pH modifiers and/or alkalizing agents, among others.

The pharmaceutically active corticosteroid a) that is present in the core is present in a concentration ranging from 0.1 0 to 10 mg and is selected from the following group: hydrocortisone, cortisone, corticosterone, prednisone, prednisolone, 6α-methyl-prednisolone, triamcinolone, betamethasone, dexamethasone, as well as their pharmaceutically acceptable salts; and/or combinations thereof.

The pharmaceutically active group belonging to non-steroidal anti-inflammatory agent b) that is present in the underlayer is present in a concentration ranging from 10 to 125 mg and is selected from the following group: aceclofenac, acetylsalicylic acid, ketoprofen, etodolac, flurbiprofen, piroxicam, indomethacin, ibuprofen, sulindac, naproxen, diclofenac, fenoprofen and their pharmaceutically acceptable salts; and/or combinations thereof.

The pharmaceutically active agent of the group belonging to non-steroidal anti-inflammatory agents c) that is present in the top layer is present in a concentration range that goes from 10 to 125 mg and is selected from the following group: aceclofenac, acetylsalicylic acid, ketoprofen, etodolac, flurbiprofen, piroxicam, indomethacin, ibuprofen, sulindac, naproxen, diclofenac, fenoprofen, and their pharmaceutically acceptable salts; and/or combinations thereof.

The diluent i) for each layer is selected from: starch, and/or pre-gelatinized starch, and/or corn starch, and/or sugar, and/or sugar compressible, and/or isomalt, and/or microcrystalline cellulose, and/or lactose, and/or sorbitol, and/or sucrose, and/or tragacanth, and/or talc, and/or trehalose, and/or xylitol, and/or the combination of at least two of the above, among others.

The binder agent ii) for each layer is selected from: acacia, and/or agar, and/or alginic acid, and/or calcium carbonate, and/or calcium lactate, and/or carbomer, and/or carboxymethyl cellulose calcium, and/or microcrystalline cellulose, and/or cellulose, and/or ceratonia, and/or chitosan, and/or copovidone, and/or dextrates, and/or dextrin, and/or dextrose, and/or ethyl cellulose, and/or gelatin, and/or glyceryl behenate, and/or guar gum, and/or hydroxyethyl cellulose, and/or hydroxymethyl cellulose, and/or low-substituted hydroxypropyl cellulose, and/or hydroxypropyl starch, and/or methylcellulose, and/or inulin, and/or lactose monohydrate, and/or magnesium aluminum silicate, and/or maltodextrin, and/or methylcellulose, and/or polycarbophil, and/or polydextrose, and/or polyethylene oxide, and/or polymethacrylates, and/or polyvinylpyrrolidone, and/or iodine, and/or sodium alginate, and/or starch, and/or pre-gelatinized starch, and/or sucrose starch, and/or polyethylene glycol succinate, and/or zein, and/or the combination of at least two of the above, among others.

The release polymer agent iii) for the nucleus and the top layer is selected from: carboxymethyl cellulose calcium, and/or carboxymetyl cellulose sodium , and/or cellulose acetate , and/or cellulose, and/or chitosan, and/or ethylcellulose, and/or gelatin, and/or hydroxyethyl cellulose, and/or hydroxymethyl cellulose, and/or hydroxypropyl cellulose, and/or hypromellose, and/or hypromellose succinate, and/or hypromellose phthalate, and/or maltodextrin, and/or methylcellulose, and/or poloxamer, and/or polydextrose, and/or poly-LD-lactic acid, and/or polyethylene oxide, and/or polymethacrylates, and/or vinyl ether/maleic anhydride), and/or polyvinyl acetate phthalate, and/or shellac, and/or zein, and/or carbomer and their derivatives, and/or the combination of at least two of the above, among others.

The disintegrating agent iii *) for the underlayer is selected from the following group: alginic acid, and/or crospovidone, and/or ion exchange resins, and/or aluminum silicate, and/or magnesium silicate, and/or microcrystalline cellulose, and/or starch, and/or sodium glycolate starch, and/or modified cellulose gum, and/or PVP, and/or sodium dodecyl sulphate, and/or corn starch, and/or rice starch, and/or cross-linked N-vinyl-2-pyrrolidone, and/or croscarmellose sodium, and/or formaldehyde-casein, and/or a combination of at least two of the above, among others.

The lubricant iv) is selected from: calcium stearate, and/or glyceryl behenate, and/or glyceryl monostearate, and/or glyceryl palmitostearate, and/or lauric acid, and/or leucine, and/or magnesium stearate, and/or maltodextrin, and/or mineral oil, and/or light mineral oil, and/or myristic acid, and/or palmitic acid, and/or polyethylene glycol, and/or polyvinyl alcohol, and/or potassium benzoate, and/or sodium chloride, and/or sodium hyaluronate, and/or sodium lauryl sulfate, and/or sodium stearyl fumarate, and/or stearic acid, and/or talc, and/or hydrogenated vegetable oil, and/or zinc stearate, and/or a combination of at least two of the above, among others.

The solvent agent v) for each layer is selected from acetone, and/or methanol, and/or ethanol, and/or absolute ethanol, and/or 80% ethanol, and/or 90% ethanol, and/or chloroform, and/or benzyl alcohol, and/or benzyl benzoate, and/or butylene glycol, and/or castor oil, and/or corn oil, and/or cottonseed oil, and/or dibutyl phthalate, and/or diethyl phthalate, and/or dimethyl phthalate, and/or dimethyl sulfoxide, and/or dimethylacetamide, and/or ethyl acetate, and/or ethyl lactate, and/or ethyl oleate, and/or glycerine, and/or glycofurol, and/or isopropyl alcohol, and/or isopropyl myristate, and/or isopropyl palmitate, and/or chain triglycerides medium, and/or mineral oil, and/or light mineral oil, and/or octyldodecanol, and/or peanut oil, and/or polyethylene glycol, and/or propylene carbonate, and/or propylene, and/or pyrrolidone, and/or safflower oil, and/or sesame oil, and/or soybean oil, and/or sunflower oil, and/or triacetin, and/or tricaprylin, and/or triethyl citrate, and/or triolein, and/or water, and/or a combination of at least two of these, among others.

The agent for pH modification is selected from: adipic acid, and/or boric acid, and/or calcium carbonate, and/or calcium lactate, and/or meglumine, and/or calcium phosphate, and/or citric acid monohydrate, and/or glycine, and/or maleic acid, and/or methionine, and/or monosodium glutamate, and/or potassium citrate, and/or sodium acetate, and/or sodium borate, and/or sodium carbonate, and/or sodium citrate dihydrate, and/or sodium hydroxide, and/or dibasic sodium phosphate, and/or monobasic sodium phosphate, and/or a combination of at least two of these, among others.

The alkalizing agent is selected from the following group: potassium citrate, and/or sodium citrate, and/or sodium hydroxide, and/or potassium hydroxide, and/or sodium bicarbonate, and/or sodium carbonate, and/or calcium bicarbonate, and/or calcium carbonate, and/or potassium bicarbonate, and/or disodium phosphate, and/or trisodium phosphate, and/or arginine, and/or talc, and/or 1-leucine, and/or the combination of at least two of these, among others.

Examples of novel pharmaceutical compositions containing the concomitant combination of a pharmaceutically active corticosteroid and a pharmaceutically active non-steroidal anti-inflammatory agent, as well as suitable excipients are also described; which present a novel three-phase release system. However, the scope of the present invention is not limited to these examples:
Example 1: Pharmaceutical composition with three-phase release system.

| **Nucleus (delayed release)** | |
|---|---|
| Component | Concentration (mg) |
| Betamethasone | O. 10-10 |
| Microcrystalline cellulose | 2.5-40 |
| Lactose | 2. 5-40 |
| Methylcellulose | 10-95 |
| Polyvinyl pyrrolidone | 1-15 |
| Magnesium stearate | 1-10 |
| Ethanol | 0. 5-4 |

| **Underlayer (immediate release)** | |
|---|---|
| Component | Concentration (mg) |
| Aceclofenac | 10-125 |
| Microcrystalline cellulose | 10-1 00 |
| Lactose | 1 0-1 00 |
| Cornstarch | 10-1 00 |
| Polyvinyl pyrrolidone | 1-50 |
| Crospovidone | 1-50 |
| Stearic acid | 1-1 5 |
| Ethanol | 0.5-4 |

| **Top layer (extended release)** | |
|---|---|
| Component | Concentration (mg) |
| Aceclofenac | 10-125 |
| Microcrystalline cellulose | 10-100 |
| Lactose | 10-100 |
| Cornstarch | 10-100 |
| Polyvinyl pyrrolidone | 1-50 |
| Methylcellulose | 10-95 |
| Stearic acid | 1-1 5 |
| Ethanol | 0.5-4 |

Example 2: Pharmaceutical composition with three-phase release system.

| **Nucleus (extended release)** | |
|---|---|
| Component | Concentration (mg) |
| Betamethasone | 0.10-10 |
| Microcrystalline cellulose | 2.5-40 |
| Lactose | 2.5-40 |
| Pre-gelatinized starch | 2.5-40 |
| Hydroxypropyl methyl cellulose | 10-95 |
| Talc | 1-15 |
| Calcium stearate | 1-10 |
| Absolute ethanol | 0.5-4 |

| **Underlayer (immediate release)** | |
|---|---|
| Component | Concentration (mg) |
| Aceclofenac | 10-1 25 |
| Microcrystalline cellulose | 10-100 |
| Lactose | 10-100 |
| Pre-gelatinized starch | 10-100 |
| Polyvinyl pyrrolidone | 1-50 |
| Crospovidone | 1-50 |
| Calcium stearate | 1-15 |
| Absolute ethanol | 0.5-4 |

| **Top layer (delayed release)** | |
|---|---|
| Component | Concentration (mg) |
| Aceclofenac | 10-1 25 |
| Microcrystalline cellulose | 10-100 |
| Lactose | 10-100 |
| Pre-gelatinized starch | 10-100 |
| Talc | 1-50 |
| Hydroxypropyl methyl cellulose | 10-95 |
| Calcium stearate | 1-15 |
| Absolute ethanol | 0.5-4 |

Furthermore, such pharmaceutical compositions with three-phase release system are characterized by having a humidity of between 2.5% and 5% in powder; a hardness of between 6 kg and 9 kg; and friability less than 1.

Process for preparing the solid oral dosage unit with three-phase release system:
For the core:
   1. Mix the pharmaceutically active corticosteroid, one or more diluents and one or more binders.
   2. Granulate the above mixture with one or more solvents, sieve, dry, and sieve through a mesh.
   3. Add and mix one or more polymeric release agents.
   4. Add and mix one or more lubricants.
   5. Compress.
For the underlayer:
   1. Mix the pharmaceutically active non-steroidal anti-inflammatory agent, one or more diluents, and one or more binders.
   2. Granulate the above mixture with one or more solvents, sieve, dry, and sieve through a mesh.
   3. Add and mix one or more disintegrating agents.
   4. Add and mix one or more lubricants.
For the top layer:
   1. Mix the pharmaceutically active non-steroidal anti-inflammatory agent, one or more diluents, and one or more binders.
   2. Granulate the above mixture with one or more solvents, sieve, dry, and sieve through a mesh.
   3. Add and mix one or more polymeric release agents.
   4. Add and mix one or more lubricants.
Tableting:
   1. Dose in each of the hoppers the nucleus, the powder for the underlayer and the powder for the top layer.
   2. Compress the powder and core with a proper punch.

Importantly, this pharmaceutical composition with a three-phase release system offers in a non-limitative way, advantages such as: an effective, efficient and safe concomitant pharmacological effect; doses of the pharmaceutically active agents are low, which prevents secondary and/or side effects to the body by oral administration of both.

## Claims

1. A pharmaceutical oral tablet, **characterized by** comprising a three-phase release system with three layers: a) a core, b) an under layer, and c) a top layer, each one comprising a pharmaceutically acceptable carrier, wherein the core comprises one corticosteroid agent, preferably betamethasone, or pharmaceutically acceptable salts thereof; the under layer comprises one non-steroidal anti-inflammatory agent, preferably aceclofenac, or pharmaceutically acceptable salts thereof; the top layer comprises one non-steroidal anti-inflammatory agent, preferably aceclofenac, or pharmaceutically acceptable salts thereof, and wherein the core has a delayed-release system or an extended release system; the under layer covers at least half of the central core and has an immediate-release system; the top layer completely covers the central core and has an extended-release system or delayed-release system; the under layer and the top layer have different release systems, and the tablet is formulated in at least one dosage unit and is useful for the treatment of body pain and inflammation of affected areas.

2. The pharmaceutical oral tablet according to claim 1, wherein the pharmaceutically active corticosteroid agent is betamethasone and is in a concentration of 0.1 to 10 mg, and the pharmaceutically active non-steroidal anti-inflammatory agent is aceclofenac and is in a concentration of 10 to 125 mg.

3. The pharmaceutical oral tablet according to claim 1, wherein the under layer comprises one or more excipients and/or pharmaceutically acceptable additives, selected from a diluent, a binder, a disintegrant, a lubricant, a solvent, a pH modifier, and an alkalizing agent.

4. The pharmaceutical oral tablet according to claim 1, comprising a three-phase release system with three layers: a) a core, b) an under layer, and c) a top layer, each one comprising a pharmaceutically acceptable carrier, wherein the core comprises one corticosteroid agent selected from betamethasone, and its pharmaceutically acceptable salts, and preferably betamethasone in an amount of 0.1 -10 mg; the under layer comprises one non-steroidal anti-inflammatory agent selected from aceclofenac, and pharmaceutically acceptable salts thereof, preferably aceclofenac in an amount of 10-125 mg; the top layer comprises one non-steroidal anti-inflammatory agent selected from aceclofenac and pharmaceutically acceptable salts thereof, preferably aceclofenac in an amount of 10-125 mg; and wherein the core has a delayed-release system or an extended-release system; the under layer covers at least half of the central core and has an immediate-release system; the top layer completely covers the central core and has an extended-release system or delayed-release system; the under layer and the top layer have different release systems, and the tablet is made in at least one dosage unity and it is useful for the treatment of body pain and inflammation of affected areas.

5. The pharmaceutical oral tablet according to claim 4, wherein the core has a delayed-release system; the under layer has an immediate-release system; and the top layer has an extended-release system.

6. The pharmaceutical oral tablet according to claim 1, wherein the core comprises a pharmaceutically acceptable carrier selected from the diluent or binder pregelatinized starch, the diluent or binder microcrystalline cellulose, the diluent lactose, and the binder polyvinylpyrrolidone, and at least one solvent selected from ethanol, absolute ethanol, 80% ethanol and 90% ethanol.

7. The pharmaceutical oral tablet according to claim 1, wherein the under layer comprises a pharmaceutically acceptable carrier selected from the diluent or binder pregelatinized starch, the diluent or binder microcrystalline cellulose, the diluent lactose, and the binder polyvinylpyrrolidone, and at least one solvent selected from ethanol, absolute ethanol, 80% ethanol and 90% ethanol.

8. The pharmaceutical oral tablet according to claim 1, wherein the top layer comprises a pharmaceutically acceptable carrier selected from the diluent or binder pregelatinized starch, the diluent or binder microcrystalline cellulose, the diluent lactose, and the binder polyvinylpyrrolidone, and at least one solvent selected from ethanol, absolute ethanol, 80% ethanol and 90% ethanol.

9. The pharmaceutical oral tablet according to claim 1, wherein the core further comprises at least one release polymer selected from calcium carboxymethyl cellulose, sodium carboxymethyl cellulose, cellulose acetate, cellulose and methylcellulose, preferably methylcellulose, and at least one lubricant selected from calcium stearate and magnesium stearate.

10. The pharmaceutical oral tablet according to claim 1, wherein the under layer further comprises at least one disintegrant selected from alginic acid and crospovidone, and at least one lubricant selected from calcium stearate and stearic acid.

11. The pharmaceutical oral tablet according to claim 1, wherein the top layer further comprises at least one release polymer selected from calcium carboxymethyl cellulose, sodium carboxymethyl cellulose, cellulose acetate, cellulose and methylcellulose, preferably methylcellulose, and at least one lubricant selected from calcium stearate, magnesium stearate and stearic acid.

12. A process for the preparation of the pharmaceutical oral tablet according to claims 1 to 11, wherein the process comprises the following production steps:
a) for the core:
i) mix the pharmaceutically active corticosteroid agent, one or more diluents and one or more binders;
ii) granulate the above mixture with one or more solvents, sieve, dry, and sieve through a mesh;
iii) add and mix one or more polymeric release agents;
iv) add and mix one or more lubricants;
v) compress the powder with a suitable punch;
b) for the under layer:
i) mix the pharmaceutically active non-steroidal anti-inflammatory agent, one or more diluents, and one or more binders;
ii) granulate the above mixture with one or more solvents, sieve, dry, and sieve through a mesh;
iii) add and mix one or more disintegrating agents;
iv) add and mix one or more lubricants;
c) for the top layer:
i) mix the pharmaceutically active non-steroidal anti-inflammatory agent, one or more diluents, and one or more binders;
ii) granulate the above mixture with one or more solvents, sieve, dry, and sieve through a mesh;
iii) add and mix one or more polymeric release agents;
iv) add and mix one or more lubricants;
d) for tableting of core (a), and powders, (b) and (c):
i) dose in independent hoppers, the core, the powder for the under layer and the powder for the top layer, to form the tablet in such a way that the core is located in the center, the under layer covers at least half of the central core, and the top layer completely covers the central core and gives the final form of the oral dosage unit;
ii) compress the powder and core with a suitable punch.

## Patentansprüche

1. Pharmazeutische orale Tablette, **gekennzeichnet durch** Umfassen eines dreiphasigen Freisetzungssystems mit drei Schichten:
a) einem Kern,
b) einer Unterschicht, und
c) einer Deckschicht,
wobei jede Schicht einen pharmazeutisch verträglichen Träger aufweist,
wobei der Kern ein Kortikosteroidmittel, vorzugsweise Betamethason, oder dessen pharmazeutisch verträgliche Salze aufweist; die Unterschicht ein nichtsteroidales entzündungshemmendes Mittel, vorzugsweise Aceclofenac, oder dessen pharmazeutisch verträgliche Salze aufweist; die Deckschicht ein nichtsteroidales entzündungshemmendes Mittel, vorzugsweise Aceclofenac, oder dessen pharmazeutisch geeignete Salze aufweist; und
wobei der Kern ein System mit verzögerter Freisetzung oder ein System mit verlängerter Freisetzung aufweist; die Unterschicht mindestens die Hälfte des zentralen Kerns bedeckt und ein System mit sofortiger Freisetzung aufweist; die Deckschicht den zentralen Kern vollständig bedeckt und ein System mit verlängerter oder verzögerter Freisetzung aufweist; die Unterschicht und die Deckschicht unterschiedliche Freisetzungssysteme aufweisen, und
wobei die Tablette in mindestens einer Dosierungseinheit formuliert ist und zur Behandlung von Körperschmerzen und Entzündungen von betroffenen Bereichen geeignet ist.

2. Pharmazeutische orale Tablette nach Anspruch 1, wobei das pharmazeutisch wirksame Kortikosteroidmittel Betamethason ist und in einer Konzentration von 0,1 bis 10 mg vorliegt, und das pharmazeutisch wirksame nichtsteroidale entzündungshemmende Mittel Aceclofenac ist und in einer Konzentration von 10 bis 125 mg vorliegt.

3. Pharmazeutische orale Tablette nach Anspruch 1, wobei die Unterschicht einen oder mehrere Hilfsstoffe und/oder pharmazeutisch verträgliche Additive aufweist, ausgewählt aus einem Verdünnungsmittel, einem Bindemittel, einem Sprengmittel, einem Gleitmittel, einem Lösungsmittel, einem pH-Modifikator und einem Alkalisierungsmittel.

4. Pharmazeutische orale Tablette nach Anspruch 1, umfassend ein dreiphasiges Freisetzungssystem mit drei Schichten:
a) einem Kern,
b) eine Unterschicht, und
c) eine Deckschicht,
wobei jede Schicht einen pharmazeutisch verträglichen Träger aufweist,
wobei der Kern ein Kortikosteroidmittel, ausgewählt aus Betamethason und dessen pharmazeutisch verträglichen Salzen, und vorzugsweise Betamethason in einer Menge von 0,1 bis 10 mg, aufweist; die Unterschicht ein nichtsteroidales entzündungshemmendes Mittel, ausgewählt aus Aceclofenac und dessen pharmazeutisch verträglichen Salzen, vorzugsweise Aceclofenac in einer Menge von 10 bis 125 mg, aufweist; die Deckschicht ein nichtsteroidales entzündungshemmendes Mittel, ausgewählt aus Aceclofenac und dessen pharmazeutisch verträglichen Salzen, vorzugsweise Aceclofenac in einer Menge von 10 bis 125 mg, aufweist;
und wobei der Kern ein System mit verzögerter Freisetzung oder ein System mit verlängerter Freisetzung aufweist; die Unterschicht mindestens die Hälfte des zentralen Kerns bedeckt und ein System mit sofortiger Freisetzung aufweist; die Deckschicht den zentralen Kern vollständig bedeckt und ein System mit verlängerter Freisetzung oder ein System mit verzögerter Freisetzung aufweist; die Unterschicht und die Deckschicht unterschiedliche Freisetzungssysteme aufweisen, und
wobei die Tablette in mindestens einer Dosierungseinheit formuliert ist und zur Behandlung von Körperschmerzen und Entzündungen von betroffenen Bereichen geeignet ist.

5. Pharmazeutische orale Tablette nach Anspruch 4, wobei der Kern ein System mit verzögerter Freisetzung, die Unterschicht ein System mit sofortiger Freisetzung und die Deckschicht ein System mit verlängerter Freisetzung aufweist

6. Pharmazeutische orale Tablette nach Anspruch 1, wobei der Kern einen pharmazeutisch verträglichen Träger, ausgewählt aus dem Verdünnungsmittel oder Bindemittel vorgelatinierter Stärke, dem Verdünnungsmittel oder Bindemittel mikrokristalline Cellulose, dem Verdünnungsmittel Lactose, und dem Bindemittel Polyvinylpyrrolidon, und mindestens ein Lösungsmittel, ausgewählt aus Ethanol, absolutem Ethanol, 80 % Ethanol und 90 % Ethanol, umfasst.

7. Pharmazeutische orale Tablette nach Anspruch 1, wobei die Unterschicht einen pharmazeutisch verträglichen Träger umfasst, ausgewählt aus dem Verdünnungsmittel oder Bindemittel vorgelatinierte Stärke, dem Verdünnungsmittel oder Bindemittel mikrokristalline Cellulose, dem Verdünnungsmittel Lactose, und dem Bindemittel Polyvinylpyrrolidon, und mindestens ein Lösungsmittel, ausgewählt aus Ethanol, absolutem Ethanol, 80 % Ethanol und 90 % Ethanol, umfasst.

8. Pharmazeutische orale Tablette nach Anspruch 1, wobei die Deckschicht einen pharmazeutisch verträglichen Träger, ausgewählt aus dem Verdünnungsmittel oder Bindemittel vorgelatinierte Stärke, dem Verdünnungsmittel oder Bindemittel mikrokristalline Cellulose, dem Verdünnungsmittel Lactose, und dem Bindemittel Polyvinylpyrrolidon, und mindestens ein Lösungsmittel, ausgewählt aus Ethanol, absolutem Ethanol, 80 % Ethanol und 90 % Ethanol, aufweist

9. Pharmazeutische orale Tablette nach Anspruch 1, wobei der Kern ferner mindestens ein Freisetzungspolymer, ausgewählt aus Calciumcarboxymethylcellulose, Natriumcarboxymethylcellulose, Celluloseacetat, Cellulose und Methylcellulose, vorzugsweise Methylcellulose, und mindestens ein Gleitmittel, ausgewählt aus Calciumstearat und Magnesiumstearat, aufweist

10. Pharmazeutische orale Tablette nach Anspruch 1, wobei die Unterschicht weiterhin mindestens ein Sprengmittel, ausgewählt aus Alginsäure und Crospovidon, und mindestens ein Gleitmittel, ausgewählt aus Calciumstearat und Stearinsäure, aufweist.

11. Pharmazeutische orale Tablette nach Anspruch 1, wobei die Deckschicht ferner mindestens ein Freisetzungspolymer, ausgewählt aus Calciumcarboxymethylcellulose, Natriumcarboxymethylcellulose, Celluloseacetat, Cellulose und Methylcellulose, vorzugsweise Methylcellulose, und mindestens ein Gleitmittel, ausgewählt aus Calciumstearat, Magnesiumstearat und Stearinsäure, aufweist

12. Verfahren zur Herstellung der pharmazeutischen oralen Tablette nach den Ansprüchen 1 bis 11, wobei das Verfahren die folgenden Herstellungsschritte aufweist:
a) für den Kern:
i) Mischen des pharmazeutisch wirksamen Kortikosteroidmittels, eines oder mehrerer Verdünnungsmittel und eines oder mehrerer Bindemittel;
ii) Granulieren der obigen Mischung mit einem oder mehreren Lösungsmitteln, Sieben, Trocknen und Sieben durch ein Sieb;
iii) Hinzufügen und Mischen eines oder mehrerer polymerer Freisetzungsmittel;
iv) Hinzufügen und Mischen eines oder mehrerer Gleitmittel;
v) Komprimieren des Pulvers mit einem geeigneten Stempel;
b) für die Unterschicht:
i) Mischen des pharmazeutisch wirksamen nichtsteroidalen entzündungshemmenden Mittels, eines oder mehrerer Verdünnungsmittel und eines oder mehrerer Bindemittel;
ii) Granulieren der obigen Mischung mit einem oder mehreren Lösungsmitteln, Sieben, Trocknen und Sieben durch ein Sieb;
iii) Hinzufügen und Mischen eines oder mehrerer Sprengmittel;
iv) Hinzufügen und Mischen eines oder mehrerer Gleitmittel;
c) für die Deckschicht:
i) Mischen des pharmazeutisch wirksamen nichtsteroidalen entzündungshemmenden Mittels, eines oder mehrerer Verdünnungsmittel und eines oder mehrerer Bindemittel;
ii) Granulieren der obigen Mischung mit einem oder mehreren Lösungsmitteln, Sieben, Trocknen und Sieben durch ein Sieb;
iii) Hinzufügen und Mischen eines oder mehrerer polymerer Freisetzungsmittel;
iv) Hinzufügen und Mischen eines oder mehrerer Gleitmittel;
d) für die Tablettierung von Kern (a) und Pulvern, (b) und (c):
i) Dosieren des Kerns, des Pulvers für die Unterschicht und des Pulvers für die Oberschicht in unabhängigen Trichtern, um die Tablette so zu formen, dass sich der Kern in der Mitte befindet, die Unterschicht mindestens die Hälfte des zentralen Kerns bedeckt und die Oberschicht den zentralen Kern vollständig bedeckt und die endgültige Form der oralen Dosierungseinheit ergibt;
ii) das Pulver und den Kern mit einem geeigneten Stempel komprimieren.

## Revendications

1. Comprimé pharmaceutique oral, **caractérisé en ce qu'**il comprend un système de libération en trois phases avec trois couches : a) un noyau, b) une couche inférieure et c) une couche supérieure, chacune comprenant un support pharmaceutiquement acceptable, dans lequel le noyau comprend un agent corticostéroïdien, de préférence de la bétaméthasone, ou des sels pharmaceutiquement acceptables de celui-ci ; la couche inférieure comprend un agent anti-inflammatoire non stéroïdien, de préférence de l'acéclofénac, ou des sels pharmaceutiquement acceptables de celui-ci ; la couche supérieure comprend un agent anti-inflammatoire non stéroïdien, de préférence de l'acéclofénac, ou des sels pharmaceutiquement acceptables de celui-ci, et le noyau a un système de libération retardée ou un système de libération prolongée ; la couche inférieure couvre au moins la moitié du noyau central et a un système de libération immédiate ; la couche supérieure couvre complètement le noyau central et a un système de libération prolongée ou un système de libération retardée ; la couche inférieure et la couche supérieure ont des systèmes de libération différents, et le comprimé est formulé en au moins une unité de dosage et est utile pour le traitement de la douleur corporelle et de l'inflammation de zones affectées.

2. Comprimé pharmaceutique oral selon la revendication 1, dans lequel l'agent corticostéroïdien pharmaceutiquement actif est la bétaméthasone et est présent à une concentration comprise entre 0,1 et 10 mg, et l'agent anti-inflammatoire non stéroïdien pharmaceutiquement actif est de l'acéclofénac et est présent à une concentration comprise entre 10 et 125 mg.

3. Comprimé pharmaceutique oral selon la revendication 1, dans lequel la couche inférieure comprend un ou plusieurs excipients et/ou des additifs pharmaceutiquement acceptables, choisis parmi un diluant, un liant, un désintégrant, un lubrifiant, un solvant, un modificateur de pH et un agent alcalinisant.

4. Comprimé pharmaceutique oral selon la revendication 1, comprenant un système de libération en trois phases avec trois couches : a) un noyau, b) une couche inférieure et c) une couche supérieure, chacune comprenant un support pharmaceutiquement acceptable, dans lequel le noyau comprend un agent corticostéroïdien choisi parmi la bétaméthasone et ses sels pharmaceutiquement acceptables, et de préférence de la bétaméthasone en une quantité comprise entre 0,1 et 10 mg ; la couche inférieure comprend un agent anti-inflammatoire non stéroïdien choisi parmi l'acéclofénac et ses sels pharmaceutiquement acceptables, de préférence de l'acéclofénac en une quantité comprise entre 10 et 125 mg; la couche supérieure comprend un agent anti-inflammatoire non stéroïdien choisi parmi l'acéclofénac et ses sels pharmaceutiquement acceptables, de préférence de l'acéclofénac en une quantité comprise entre 10 et 125 mg ; et le noyau a un système de libération retardée ou un système de libération prolongée ; la couche inférieure couvre au moins la moitié du noyau central et a un système de libération immédiate ; la couche supérieure couvre complètement le noyau central et a un système de libération prolongée ou un système de libération retardée ; la couche inférieure et la couche supérieure ont des systèmes de libération différents, et le comprimé est formulé en au moins une unité de dosage et il est utile pour le traitement de la douleur corporelle et de l'inflammation de zones affectées.

5. Comprimé pharmaceutique oral selon la revendication 4, dans lequel le noyau a un système de libération retardée ; la couche inférieure a un système de libération immédiate ; et la couche supérieure a un système de libération prolongée.

6. Comprimé pharmaceutique oral selon la revendication 1, dans lequel le noyau comprend un support pharmaceutiquement acceptable choisi parmi le diluant ou le liant amidon prégélatinisé, le diluant ou le liant cellulose microcristalline, le diluant lactose et le liant polyvinylpyrrolidone, et au moins un solvant choisi parmi l'éthanol, l'éthanol absolu, l'éthanol à 80% et l'éthanol à 90%.

7. Comprimé pharmaceutique oral selon la revendication 1, dans lequel la couche inférieure comprend un support pharmaceutiquement acceptable choisi parmi le diluant ou le liant amidon prégélatinisé, le diluant ou le liant cellulose microcristalline, le diluant lactose, et le liant polyvinylpyrrolidone, et au moins un solvant choisi parmi l'éthanol, l'éthanol absolu, l'éthanol à 80% et l'éthanol à 90%.

8. Comprimé pharmaceutique oral selon la revendication 1, dans lequel la couche supérieure comprend un support pharmaceutiquement acceptable choisi parmi le diluant ou le liant amidon prégélatinisé, le diluant ou le liant cellulose microcristalline, le diluant lactose, et le liant polyvinylpyrrolidone, et au moins un solvant choisi parmi l'éthanol, l'éthanol absolu, l'éthanol à 80% et l'éthanol à 90%.

9. Comprimé pharmaceutique oral selon la revendication 1, dans lequel le noyau comprend en outre au moins un polymère de libération choisi parmi la carboxyméthylcellulose de calcium, la carboxyméthylcellulose de sodium, l'acétate de cellulose, la cellulose et la méthylcellulose, de préférence la méthylcellulose, et au moins un lubrifiant choisi parmi le stéarate de calcium et le stéarate de magnésium.

10. Comprimé pharmaceutique oral selon la revendication 1, dans lequel la couche inférieure comprend en outre au moins un désintégrant choisi parmi l'acide alginique et la crospovidone, et au moins un lubrifiant choisi parmi le stéarate de calcium et l'acide stéarique.

11. Comprimé pharmaceutique oral selon la revendication 1, dans lequel la couche supérieure comprend en outre au moins un polymère de libération choisi parmi la carboxyméthylcellulose de calcium, la carboxyméthylcellulose de sodium, l'acétate de cellulose, la cellulose et la méthylcellulose, de préférence la méthylcellulose, et au moins un lubrifiant choisi parmi le stéarate de calcium, le stéarate de magnésium et l'acide stéarique.

12. Procédé de préparation du comprimé pharmaceutique oral selon les revendications 1 à 11, dans lequel le procédé comprend les étapes de production suivantes :
a) Pour le noyau :
i) Mélanger l'agent corticostéroïdien pharmaceutiquement actif, un ou plusieurs diluants et un ou plusieurs liants ;
ii) Granuler le mélange ci-dessus avec un ou plusieurs solvants, tamiser, sécher et passer au tamis à maille ;
iii) Ajouter et mélanger un ou plusieurs agents de libération polymériques;
iv) Ajouter et mélanger un ou plusieurs lubrifiants ;
v) Comprimer la poudre à l'aide d'un poinçon approprié ;
b) Pour la couche inférieure :
i) Mélanger l'agent anti-inflammatoire non stéroïdien pharmaceutiquement actif, un ou plusieurs diluants et un ou plusieurs liants ;
ii) Granuler le mélange ci-dessus avec un ou plusieurs solvants, tamiser, sécher et passer au tamis à maille ;
iii) Ajouter et mélanger un ou plusieurs agents désintégrant ;
iv) Ajouter et mélanger un ou plusieurs lubrifiants ;
c) Pour la couche supérieure :
i) Mélanger l'agent anti-inflammatoire non stéroïdien pharmaceutiquement actif, un ou plusieurs diluants et un ou plusieurs liants ;
ii) Granuler le mélange ci-dessus avec un ou plusieurs solvants, tamiser, sécher et passer au tamis à maille ;
iii) Ajouter et mélanger un ou plusieurs agents de libération polymériques;
iv) Ajouter et mélanger un ou plusieurs lubrifiants ;
d) Pour comprimer le noyau (a), et les poudres, (b) et (c) :
i) Doser dans des trémies indépendantes, le noyau, la poudre pour la couche inférieure et la poudre pour la couche supérieure, pour former le comprimé de sorte que le noyau soit situé au centre, la couche inférieure recouvre au moins la moitié du noyau central et la couche supérieure recouvre complètement le noyau central et donne la forme finale de l'unité de dosage orale ;
ii) Comprimer la poudre et le noyau à l'aide d'un poinçon approprié.
